# EUROPEAN PATENT APPLICATION

(11) **EP 4 793 357 A1**
(43) Date of publication of application: **19.08.2026**
(21) Application number: 24877327.7
(22) Date of filing: 15.10.2024
(51) Int. Cl.: C12P 7/52, A23L 11/00, A23L 11/65, A23L 33/105, C12N 1/20, C12P 7/56

(54) **AGENT FOR ENHANCING BUTYRIC ACID OR LACTIC ACID PRODUCTION**

(30) Priority: 13.10.2023 JP 2023177849
(71) Applicant: Kikkoman Corporation, Noda-shi, Chiba 278-8601 (JP); National University Corporation Chiba University, Chiba-shi, Chiba 263-8522 (JP)
(72) Inventor: HAYASHI, Kanako, Noda-shi, Chiba 278-8601 (JP); GOTO, Yoshiyuki, Chiba-shi, Chiba 260-8670 (JP)
(74) Representative: Mewburn Ellis LLP
(86) International application number: PCT/JP2024/036717
(87) International publication number: WO 2025/079733

(57) **Abstract**

A butyric acid or lactic acid production enhancer containing soy milk.

## Description

### Technical Field

The present invention relates to a butyric acid or lactic acid production enhancer. The present invention also relates to a method for promoting growth of butyrate-producing bacteria.

### Background Art

Short-chain fatty acids such as butyric acid, acetic acid and propionic acid are known to have various positive effects on the bowel. For example, Non Patent Literature 1 indicates that short-chain fatty acids maintain the barrier function of the bowel, reduce pH of the inside of the bowel to suppress proliferation of pathogenic bacteria, and have an anti-inflammatory effect, an immunomodulatory function, and an effect of suppressing obesity by increasing insulin sensitivity and improving maintenance of a feeling of fullness, for example.

Among the positive effects of short-chain fatty acid on the bowel, the maintenance of the barrier function of the bowel is very important. The bowel has a function of absorbing substances necessary for maintaining living organisms, such as water and nutrient components, as well as a function of appropriately eliminating unnecessary substances such as detrimental substances and pathogenic cells, and hence an important role as a barrier that restricts entry of foreign matter. If the barrier function of the bowel is lost and the bowel tract is damaged, foreign matter enters into mucosal membranes, triggers an excessive immune reaction, and causes inflammation, and further, the foreign matter joins the bloodstream, which leads to a state of systemic inflammation.

Non Patent Literature 2 indicates that a bowel in a state where the bowel is perforated, so that leakage into the blood occurs is called a leaky gut, and the leaky gut is considered a problem because it causes chronic inflammation. Therefore, the barrier function of the bowel is essential for animals to maintain their health.

Here, Non Patent Literature 1 and Non Patent Literature 2 indicate that among short-chain fatty acids, butyric acid particularly enhances the barrier function of the bowel by increasing tight junction, and producing mucin and antimicrobial peptides. Therefore, butyric acid is expected to restrict passage of microorganisms and foreign matter into the lamina propria, and prevent the leaky gut.

Further, Non Patent Literature 3 indicates that butyric acid is essential for differentiation of regulatory T cells (Treg) that are effective for suppression of allergy and inflammatory bowel diseases (IBDs), and Non Patent Literature 4 indicates that consumption of oxygen in the bowel increases when butyric acid is metabolized by bowel tract epithelial cells. Therefore, butyric acid is expected to not only suppress IBD and enhance the barrier function of the bowel, but also have an effect of maintaining the immune response in the bowel, and an effect of improving bowel microbiota by maintaining an anaerobic condition in the bowel to increasing the number of beneficial bacteria such as bifidobacteria.

Thus, in the bowel, butyric acid plays various important roles such as enhancement of the barrier function of the bowel.

Here, dietary fibers are consumed by bacteria in the bowel, or fructose, oligofructose or inulin is consumed by butyrate-producing bacteria such as Eubacterium rectale (currently called Agathobacter rectalis) to produce butyric acid (see Non Patent Literature 5).

Among butyrate-producing bacteria, Agathobacter rectalis settles on mucous layers covering the bowel tract epithelium, and thus produces butyric acid near bowel tract epithelial cells, so that a physiological action such as enhancement of the barrier function of the bowel by butyric acid can be efficiently exhibited (see Non Patent Literature 6). Since in IBD patients, a decreased amount of microorganism groups including Agathobacter rectalis is confirmed, involvement of Agathobacter rectalis in suppression of IBD has been suggested (see Non Patent Literature 7).

In the past, it has been disclosed that an agent for enhancing the proportion of lactic acid bacteria in bacteria in the bowel and the diversity of bacterial flora in the bowel, which contains soy isoflavone as an active ingredient enhances the number of butyrate-producing bacteria (Patent Literature 1), and a composition containing soy pulp proliferates butyrate-producing bacteria (Patent Literature 2).

### Citation List

### Patent Literature

Patent Literature 1: Japanese Patent Laid Open No. 2020-158439
Patent Literature 2: Japanese Patent Laid Open No. 2020-156420

### Non-Patent Literature

Non-Patent Literature 1: Riviere et al. Bifidobacteria and Butyrate-Producing Colon Bacteria: Importance and Strategies for Their Stimulation in the Human Gut. Frontiers in Microbiology. 2016, 7, Article 979.
Non-Patent Literature 2: KOBAYASHI Kyosuke/ASAMI Yukio, Effect of Yogurt on Intestinal Barrier Function. Jpn. J. Food Microbiol. 2019, 36 (1), 32-35.
Non-Patent Literature 3: Furusawa et al. Commensal microbe-derived butyrate induces the differentiation of colonic regulatory T cells. Nature. 2013, 504, 446-450.
Non-Patent Literature 4: SUZUKI Takuya. Role of intestinal tight junction barrier and the regulation by dietary factors. Milk Science. 2020, 69 (3), 180-186.
Non-Patent Literature 5: F. Moens & De Vuyst. Inulin-type fructan degradation capacity of Clostridium cluster IV and XIVa butyrate-producing colon bacteria and their associated metabolic outcomes. Beneficial Microbes. 2017, 8(3), 473-490.
Non-Patent Literature 6: P Van den Abbeele et al. Butyrate-producing Clostridium cluster XIVa species specifically colonize mucins in an in vitro gut model. The ISME Journal. 2013, 7, 949-961.
Non-Patent Literature 7: Heinken et al. Systematic assessment of secondary bile acid metabolism in gut microbes reveals distinct metabolic capabilities in inflammatory bowel disease. Microbiome. 2019, 7, Article 75.

### Summary of Invention

### Technical Problem

A problem to be solved by the present invention is to provide a novel component capable of enhancing the production of butyric acid or lactic acid.

### Solution to Problem

The present inventors have conducted intensive studies for solving the above problems, and resultantly discovered that soy milk enhances the production of butyric acid and lactic acid, leading to completion of the present invention.

That is, the present invention is as follows.
(1) A butyric acid or lactic acid production enhancer comprising soy milk.
(2) The butyric acid or lactic acid production enhancer according to (1), comprising a polysaccharide derived from soy milk.
(3) A food composition comprising the butyric acid or lactic acid production enhancer according to (1) or (2).
(4) A food composition for enhancing the production of butyric acid or lactic acid, comprising soy milk.
(5) A method for enhancing the production of butyric acid or lactic acid using soy milk.
(6) A growth promoter for butyrate-producing bacteria, comprising soy milk.
(7) The growth promoter for butyrate-producing bacteria according to (6), comprising a polysaccharide derived from soy milk.
(8) A food composition comprising the growth promoter for butyrate-producing bacteria according to (6) or (7).
(9) A food composition for promoting growth of butyrate-producing bacteria, comprising soy milk.
(10) A method for promoting growth of butyrate-producing bacteria using soy milk.
   In the respective aspects above, the butyrate-producing bacteria may be Agathobacter rectalis.
   The present invention may be as follows.
(11) Soy milk for use in enhancing the production of butyric acid or lactic acid.
(12) A food composition comprising soy milk for use in enhancing the production of butyrate-producing bacteria or lactic acid.
(13) Use of soy milk to enhance the production of butyric acid or lactic acid.
(14) Use of a food composition comprising soy milk to enhance the production of butyric acid or lactic acid.
(15) Use of soy milk in manufacturing of a butyric acid or lactic acid production enhancer.
(16) Use of soy milk in manufacturing of a food composition for enhancing the production of butyric acid or lactic acid.

In (11) to (16) above,
a polysaccharide derived from soy milk may be contained, and/or
the butyrate-producing bacteria may be Agathobacter rectalis.

The present invention may be as follows.
(21) Soy milk for use in promotion of growth of butyrate-producing bacteria.
(22) A food composition comprising soy milk for use in promotion of growth of butyrate-producing bacteria.
(23) Use of soy milk to promote growth of butyrate-producing bacteria.
(24) Use of a food composition comprising soy milk to promote growth of butyrate-producing bacteria.
(25) Use of soy milk in manufacturing of a growth promoter for butyrate-producing bacteria.
(26) Use of soy milk in manufacturing of a food composition for promotion of growth of butyrate-producing bacteria.

In (21) to (26) above,
Soy Milk is preferably used,
a polysaccharide derived from soy milk may be contained, and/or
the butyrate-producing bacteria may be Agathobacter rectalis.

The invention may include a therapeutic invention, but may include a non-therapeutic invention. That is, an invention of a therapeutic or non-therapeutic method may be included, an invention of therapeutic or non-therapeutic use may be included, and an invention of a therapeutic or non-therapeutic thing may be included.

### Advantageous Effect of Invention

According to the present invention, a novel component capable of enhancing the production of butyric acid or lactic acid can also be provided.

### Description of Embodiments

Hereinafter, modes for carrying out the present invention (hereinafter, referred to as "the present embodiment") will be described in detail. Note that the present invention is not limited to the present embodiment below.

The present embodiment provides a butyric acid or lactic acid production enhancer containing soy milk.

In the present embodiment, the "soy milk" is a drink which contains a soy milk liquid, and is obtained by eluting protein and other components from soy (whole soy, defatted soy, powdered soy or the like) by hot water, and removing fibrous materials, and examples thereof include soy milks (soy milk, adjusted soy milk and soy milk drinks) described in Quality Labeling Standard for Soy Milk stipulated by Consumer Affairs Agency, as well as soy milks other than those described in Quality Labeling Standard for Soy Milk. The soy milk in the present embodiment may contain additional materials (for example, animal and plant fat/oil, seasoning agents such as a sweetener and salt, flavor materials such as fruit juice, vegetable juice, coffee and cocoa, an emulsifier, a starch adhesive, and a flavoring agent). Further, the soy milk according to the present embodiment includes a so-called a soy liquid preparation, which are prepared by suspending, dissolving, or homogenizing powdered dehulled soybeans or powdered whole soybeans in water, and soy drink made from the soy liquid preparation. The soy milk may be sterilized for long-term storage, and hermetically sealed and packaged in a state where commercial sterility is maintained.

The method for manufacturing soy milk is not particularly limited. The soy milk can be manufactured by a method known to those skilled in the art, for example, immersing soy in water overnight, followed by grinding with a grinder. The soy may be used after being pretreated by roasting or the like. As the soy milk, soy milk that is commonly available in the market may be used.

In the case where the final product is sol milk other than plain soy milk, various additives used for common soy milk drink may be added. Examples of the additive include saccharides such as sugar, salt, calcium lactate, emulsifiers, starch adhesives such as carrageenan, flavoring agents, coloring agents, fat/oil, preservatives, antioxidants, emulsifiers, spice, various extracts/pastes, protein and degradation products thereof, organic acids, organic acid compounds, starch, and stabilizers.

In relation to the soy solid content amount, Japanese Agricultural Standards defines soy milk as having a soy solid content of 8.0% or more, adjusted soy milk as having a soy solid content of 6% or more, and soy milk drink as having a soy solid content of 4% or more, all of which may be used as soy milk in the present embodiment.

In the present embodiment, soy milk may contain a polysaccharide. In this case, the present embodiment also provides a butyric acid or lactic acid production enhancer containing a polysaccharide derived from soy milk.

The polysaccharide derived from soy milk can be analyzed by a method known to those skilled in the art, and can be analyzed by, for example, Fourier transform infrared spectroscopy (FTIR) or an HPLC method.

The polysaccharide is a saccharide formed by large number of monosaccharide molecules bound together via a glycoside bond. The polysaccharide in the present embodiment may have a molecular weight of more than 500 Da. The polysaccharide derived from soy milk is not particularly limited, but may be, for example, a polysaccharide that is understood by referring to Japanese Patent Laid-Open No. 2012-036100. The polysaccharide derived from soy milk may contain polygalacturonic acid as a main component. Further, from the constituent saccharide composition of the polysaccharide derived from soy (NAKAMURA Akihiro, Development of Soybean Soluble Polysaccharide Derived from "Okara", and Application as a Functional Food Ingredient, Journal of Japanese Society for Food Science and Technology, 2011, 58(11), 559-566, or Japanese Patent Laid Open No. 2012-036100, or KIKUCHI Tadaaki, Changes of Soybean Cell Wall Polysaccharides during Soy Sauce Fermentation, Journal of Japan Society for Bioscience, Biotechnology and Agrochemistry, 1976, 50(6), 273-277), constituent saccharides of the polysaccharide in the present embodiment may contain galacturonic acid, galactose, arabinose, rhamnose, fucose, xylose, glucose, mannose and ribose. A part of arabinogalactan composed of arabinose and galactose that are constituent saccharides may be bound to polygalacturonic acid that is a polysaccharide included in the present embodiment.

The polysaccharide derived from soy milk is not particularly limited, but may be, for example, a polysaccharide that is understood by referring to Japanese Patent Laid Open No. 2012-036100.

As a mode of the present embodiment, for enhancing the amount of butyric acid in the bowel, it is important that polysaccharides such as dietary fibers are metabolized by butyrate-producing bacteria and the like to enhance the number of butyrate-producing bacteria. As a result of the enhanced butyrate-producing bacteria, the production of butyric acid can be expected to enhance. That is, it can be expected that metabolism of polysaccharides such as dietary fibers by butyrate-producing bacteria and the like promotes growth of butyrate-producing bacteria and the like, and the enhanced number of butyrate-producing bacteria and the like leads to an enhance in the production of butyric acid and the like. In the present embodiment, soy milk may contain a polysaccharide, and thus the polysaccharide is metabolized by butyrate-producing bacteria and the like to produce butyric acid and the like. As a result of metabolizing the polysaccharide to enhance the number of butyrate-producing bacteria and the like that produce butyric acid and the like, the amount of butyric acid in the bowel can be expected to enhance, and the physiological functions of butyric acid and the like are expected to be exhibited in the bowel.

The butyric acid or lactic acid production enhancer in the present embodiment, which contains soy milk, thus can enhance the production of butyric acid or lactic acid.

An enhanced production of butyric acid or lactic acid may be evaluated based on the production of butyric acid or lactic acid by butyrate-producing bacteria. That is, when the production of butyric acid or lactic acid by butyrate-producing bacteria is larger in the case where soy milk is given to butyrate-producing bacteria than in the case where soy milk is not given to butyrate-producing bacteria, it can be evaluated that the production enhances. For example, when the amount of butyric acid or lactic acid that one cell of butyrate-producing bacteria produces is larger in the case where soy milk is given to butyrate-producing bacteria, it can be evaluated that the production enhances. For example, when the number of butyrate-producing bacteria is larger and the production of butyric acid or lactic acid by butyrate-producing bacteria is accordingly larger in the case where soy milk is given to butyrate-producing bacteria, it can be evaluated that the production enhances. Further, when the amount of butyric acid or lactic acid that one cell of butyrate-producing bacteria produces is larger, and the number of butyrate-producing bacteria is larger in the case where soy milk is given to the butyrate-producing bacteria, it can be evaluated that the production enhances.

The production of butyric acid or lactic acid can be measured by a method known to those skilled in the art, and can be measured by, for example, a HPLC method.

The butyric acid or lactic acid production enhancer in the present embodiment may enhance the production of both butyric acid and lactic acid.

The butyrate-producing bacteria are not particularly limited, but examples thereof include Agathobacter rectalis, Faecalibacterium prausnitzii, Clostridium methylpentosum, Clostridium leptum, Clostridium sporosphaeroides, Clostridium cellulosi, Clostridium viride, Roseburia intestinalis, Roseburia inulinivorans, Roseburia faecis, Roseburia hominis, Eubacterium hallii, Anaerostipes caccae, Coprococcus comes, Coprococcus eutactus, Anaerostipes hadrus, Clostridium coccoides, Clostridium celerecrescens, Clostridium boltei, Clostridium oroticum, Clostridium nexile, Clostridium scindens, Clostridium butyricum, Blautia wexlerae, with Agathobacter rectalis being preferred.

Agathobacter rectalis that is preferred as butyrate-producing bacteria will be described as an example. Agathobacter rectalis has 52 glycosidases in a genome, which include, for example, βfructofuranosidase, α-arabinofuranosidase, β-xylosidase, exooligoxylanase, α-amylase, α- and β-glucosidase, α- and β-galactosidase, and cellulase. Other butyrate-producing bacteria also contain similar enzyme groups. Agathobacter rectalis contains a larger number of types of polysaccharide degradation enzymes over other butyrate-producing bacteria, thus metabolize polysaccharides, is expected to have high butyric acid production ability, and can be more preferred in the present invention. In the present embodiment, since soy milk may contain a polysaccharide, the soy milk may be capable of enhancing the production of butyric acid or lactic acid.

In the present embodiment, the butyric acid is a linear saturated carboxylic acid having 4 carbon atoms, and may be n-butyric acid, or isobutyric acid. In the present embodiment, the lactic acid is an α-hydroxylic acid having asymmetric carbon, and may be D-lactic acid or L-lactic acid, or may include both.

The aspect of the butyric acid or lactic acid production enhancer according to the present embodiment is also applied to other embodiments which will be described below.

As another embodiment, a method for enhancing the production of butyric acid or lactic acid using soy milk is also provided.

When the method for enhancing the production of butyric acid or lactic acid is carried out for enhancing the amount of butyric acid in the body (preferably in the bowel), or carried out in production of butyric acid by butyrate-producing bacteria, for example, the production of butyric acid by butyrate-producing bacteria can be enhanced. In the case where the method for enhancing the production of butyric acid or lactic acid is carried out for enhancing the amount of butyric acid in the body, the method may include a process of administering the butyric acid or lactic acid production enhancer in the present embodiment to a target. The administration target is preferably a mammal, more preferably a human, a monkey, a cat, a pig, a horse, a cattle, a mouse, a rat, a guinea pig, a dog or a rabbit, and further more preferably a human. The dosage amount and the administration frequency of the butyric acid or lactic acid production enhancer may be appropriately set according to a route of administration, and various conditions such as an age, a body weight and a symptom of the administration target.

As another embodiment, a food composition containing butyric acid or lactic acid production enhancer is also provided. That is, a food composition containing soy milk that enhances the production of butyric acid or lactic acid is provided.

When a food composition contains a butyric acid or lactic acid production enhancer, butyrate-producing bacteria exist in the food composition, or the food composition exists in an environment where butyrate-producing bacteria exist, and thus, a function of enhancing the production of butyric acid or lactic acid by sot milk is exhibited. The function may be exhibited directly by the butyric acid or lactic acid production enhancer, or the function may be exhibited through a food composition into which the butyric acid or lactic acid production enhancer is incorporated.

In the present embodiment, the food composition may be a food composition for enhancing the production of butyric acid or lactic acid, which contains butyric acid or lactic acid. The food composition containing a butyric acid or lactic acid production enhancer may be a food composition for enhancing the production of butyric acid or lactic acid, which contains a butyric acid or lactic acid production enhancer.

As another embodiment, a growth promoter for butyrate-producing bacteria, which contains soy milk is provided.

The growth promoter for butyrate-producing bacteria, which contains soy milk, thus can promote growth of butyrate-producing bacteria.

Promoted growth of butyrate-producing bacteria may be evaluated based on proliferation of butyrate-producing bacteria. That is, when the number of butyrate-producing bacteria is larger in the case where soy milk is given to butyrate-producing bacteria than in the case where soy milk is not given to butyrate-producing bacteria, it can be evaluated that growth of butyrate-producing bacteria is promoted.

The proliferation of butyrate-producing bacteria can be evaluated by a method known to those skilled in the art, and can be evaluated by, for example, measuring the value of OD₆₀₀.

As another embodiment, a method for promoting growth of butyrate-producing bacteria using soy milk is also provided.

When the method for promoting growth of butyrate-producing bacteria is carried out for enhancing the number of butyrate-producing bacteria in the body (preferably in the bowel), or carried out in production of butyric acid by butyrate-producing bacteria, for example, proliferation of butyrate-producing bacteria can be promoted to enhance the production of butyric acid by butyrate-producing bacteria. In the case where the method for promoting growth of butyrate-producing bacteria is carried out for enhancing the number of butyrate-producing bacteria in the body, the method may include a process of administering a growth promoter for butyrate-producing bacteria in the present embodiment to a target. The administration target is preferably a mammal, more preferably a human, a monkey, a cat, a pig, a horse, a cattle, a mouse, a rat, a guinea pig, a dog or a rabbit, and further more preferably a human. The dosage amount and the administration frequency of the growth promoter for butyrate-producing bacteria may be appropriately set according to a route of administration, and various conditions such as an age, a body weight and a symptom of the administration target.

In the present specification, the method includes a therapeutic method and a non-therapeutic method, and the use includes therapeutic use and non-therapeutic use.

As another embodiment, the food composition containing a butyric acid or lactic acid production enhancer may be understood as a food composition containing a growth promoter for butyrate-producing bacteria. That is, the food composition containing soy milk may be a food composition containing a butyric acid or lactic acid production enhancer, a food composition containing a growth promoter for butyrate-producing bacteria, or both.

In the present embodiment, the food composition may be a food composition for promoting growth of butyrate-producing bacteria, which contains butyric acid or lactic acid. The food composition containing a growth promoter for butyrate-producing bacteria may be a food composition for promoting growth of butyrate-producing bacteria, which contains a butyric acid or lactic acid production enhancer.

The form of the butyric acid or lactic acid production enhancer, or the growth promoter for butyrate-producing bacteria is not particularly limited, but may be in a dosage form of an oral preparation such as a tablet, a capsule, a granule, a subtle granule, a powder, a solution, a syrup, a chewable or a lozenge, an ointment, a gel, a cream, an injection, a sublingual formulation, an inhalant, a suppository or the like. For these dosage forms, heretofore known additives can be used on manufacturing. Although there is no particular limitation, for example, an excipient, a binder, a diluent, a disintegrant, a buffer, a thickener, a stabilizer, an emulsifier, a dispersant, a suspending agent and/or an antiseptic agent which are known in the industry may be used as necessary.

The butyric acid or lactic acid production enhancer, or the growth promoter for butyrate-producing bacteria may be blended in food, feed, medicaments and the like.

The content of soy milk in the butyric acid or lactic acid production enhancer or the growth promoter for butyrate-producing bacteria is not limited as long as a relevant function can be exhibited. The content is preferably 0.1 mass% or more, and may be, for example, 0.1 mass%, 0.2 mass%, 0.5 mass%, 1.0 mass%, 2.0 mass%, 3.0 mass%, 4.0 mass%, 5.0 mass%, or 10 mass% or more, based on the total amount of the butyric acid or lactic acid production enhancer or the growth promoter for butyrate-producing bacteria.

The food composition can be made into, for example, a form suitable for a mode of use, and may be in a dosage form of an oral preparation such as a tablet, a capsule, a granule, a subtle granule, a powder, a solution, a syrup, a chewable or a lozenge, an ointment, a gel, a cream, an injection, a sublingual formulation, an inhalant, a suppository or the like although there is no particular limitation. Although there is no particular limitation, for example, the food composition can be made into an edible form such as a solid form, a liquid form, a granulated form, a granular form, a powder form, a capsule form, a cream form, and a paste form or a jelly form. When the food composition is made into a capsule form, it may be covered with one or more layers of film as necessary. For making the food composition into such a dosage form or form, heretofore known additives can be used for manufacturing. Although there is no particular limitation, for example, an excipient, a binder, a diluent, a disintegrant, a buffer, a thickener, a stabilizer, an emulsifier, a dispersant, a suspending agent and/or an antiseptic agent which are known in the food industry may be used as necessary.

The food composition is not particularly limited, but examples thereof include food and drink, health food, functional food, food for specified health use, food with nutrient function claims, health supplements, and supplements.

Examples of the food and drink include beverages, food, seasoning agents, and food additives, specific examples of the beverage include soft drink, fruit juice drink, carbonated drink, milk beverages, alcohol drink, sports drink, and nutritional drink, and specific examples of the food include bread, noodles, rice, soup, prepared food, bean curd, dairy products, and confectionery. Specific examples of the seasoning agent include those used for applications such as bean paste, soy sauce or dressing.

To the food composition, for example, a coloring agent, a flavoring agent, a sweetener, a bittering agent, a sour agent, a preservative, an antioxidant, a pH adjuster, and/or a thickening agent may be added as necessary.

The food composition may be provided after being sterilized by heating, and may be provided in the form of being sealed in a bag or a container. The food composition may be provided in the form of being labeled as "enhance barrier function of bowel", "enhance butyrate-producing bacteria in bowel", "grow butyrate-producing bacteria in bowel" or "suited to people who are concerned about bowel condition".

The food composition may preferably contain an arbitrary amount of a butyric acid or lactic acid production enhancer, or a growth promoter for butyrate-producing bacteria as long as a relevant function can be exhibited.

### Examples

Hereinafter, the present invention will be described in more detail by showing specific examples. Note that the present invention is not limited to these examples.

### <Example 1> Evaluation of effect of soy milk on proliferation of butyrate-producing bacteria and production of n-butyric acid and lactic acid

### (1) Preparation of modified YCFA medium

YCFA medium for use in culture of butyrate-producing bacteria was prepared by following Tables 1-1 to 1-6 below. The prepared YCFA medium was adjusted to a pH of 7.45 with 10 N NaOH, and then sterilized in an autoclave at 121°C for 20 minutes. Thereafter, the medium sterilized in the autoclave was brought back to room temperature, and 0.5 mL of Vitamin Solution II (Table 1-7) was added.

Subsequently, a preculture solution obtained by culturing butyrate-producing bacteria Agathobacter rectalis JCM 17463^{T} strain (hereinafter, referred to as A. rectalis) purchased from Riken BioResource Research Center was inoculated in the YCFA medium at 2% (v/v), and subjected to stationary culture at 37°C for 16 hours under a constant hydrogen concentration condition in an oxygen-free anaerobic chamber (manufactured by Coy Laboratory Products, Inc.). The resulting culture solution was centrifuged at 7,500 rpm (5,100 × g) for 10 minutes, and the supernatant was sterilized by filtration twice with a 0.20 µm Minisart (registered trademark) syringe filter (manufactured by Sartorius AG) to obtain modified YCFA medium.

**Table 1-1 YCFA medium 500 mL**

| [Table 1-1] | |
|---|---|
| Name of component | Weight or volume |
| Tryptone | 5.00 g |
| Yeast extract | 1.25 g |
| NaHCO₃ | 2.00 g |
| Glucose | 1.00 g |
| Maltose | 1.00 g |
| Cellobiose | 1.00 g |
| L-Cysteine·HCl·H₂O | 0.50 g |
| Resazurin | 0.5 mg |
| Mineral solution I | 75.0 mL |
| Mineral solution II | 75.0 mL |
| VFA mix | 3.1 mL |
| Hemin solution | 5.0 mL |
| Vitamin solution I | 0.5 mL |
| Distilled water | 330.0 mL |

**Table 1-2 Mineral solution I**

| [Table 1-2] | |
|---|---|
| Name of component | Weight or volume |
| K₂HPO₄ | 0.225 g |
| Distilled water | 75.00 mL |

**Table 1-3 Mineral solution II**

| [Table 1-3] | |
|---|---|
| Name of component | Weight or volume |
| KH₂PO₄ | 0.225 g |
| (NH₄)₂SO₄ | 0.450 g |
| NaCl | 0.450 g |
| MgSO₄·7H₂O | 0.045 g |
| CaCl₂·2H₂O | 0.045 g |
| Distilled water | 75.00 mL |

**Table 1-4 VFA (volatile fatty acid) mix**

| [Table 1-4] | |
|---|---|
| Name of component | Volume |
| Acetic acid | 17.0 mL |
| Propionic acid | 6.0 mL |
| n-Valeric acid | 1.0 mL |
| iso-Valeric acid | 1.0 mL |
| iso-Butyric acid | 1.0 mL |
| 10N NaOH | 26.0 mL |

**Table 1-5 Hemin solution**

| [Table 1-5] | |
|---|---|
| Name of component | Weight or volume |
| potassium hydroxide | 0.14 g |
| Hemin | 0.05 g |
| Ethanol | 12.5 mL |
| Distilled water | 37.5 mL |

**Table 1-6 Vitamin Solution I**

| [Table 1-6] | |
|---|---|
| Name of component | Weight or volume |
| Biotin | 0.5 mg |
| Vitamin B12 | 0.5 mg |
| p-Aminobenzoic acid | 1.5 mg |
| Folic acid | 2.5 mg |
| Pyridoxine·HCl | 7.5 mg |
| Distilled water | 50.0 mL |

**Table 1-7 Vitamin Solution II**

| [Table 1-7] | |
|---|---|
| Name of component | Weight or volume |
| Thiamine·HCl | 2.5 mg |
| Riboflavin | 2.5 mg |
| Distilled water | 50.0 mL |

### (2) Evaluation of effect of soy milk on proliferation of A. rectalis and production of n-butyric acid and lactic acid

A. rectalis stored at -80°C as a glycerol stock was thawed in an anaerobic chamber (manufactured by Coy Laboratory Products, Inc.), diluted 5-fold with the YCFA medium, and subjected to stationary culture overnight at 37°C under a constant hydrogen concentration condition to perform restoration culture. The resulting restoration culture solution was diluted 10-fold with the YCFA medium, and subjected to successive culture, and the resulting culture solution was diluted with modified YCFA medium so that OD₆₀₀ was within a range of 0.15 to 0.25, thereby preparing a successive culture solution.

The successive culture solution was inoculated in an amount of 50 µL in 950 µL of modified YCFA medium containing filtered plain soy milk (KIKKOMAN CORPORATION) in a ratio of 5% (v/v), or modified YCFA medium free of plain soy milk as a control, and subjected to stationary culture at 37°C for 15 hours under a constant hydrogen concentration condition. After the stationary culture, OD₆₀₀ of the culture solution was measured.

The supernatant of the culture solution subjected to stationary culture was centrifuged at 15,000 rpm (204 × 100 g) and 4°C for 10 minutes. The centrifugal supernatant was sterilized by filtration with 0.22 µm Millex (registered trademark) - GV, PVDF (manufactured by Merck KGaA), and added to 500 µL in volume of Amicon (registered trademark) Ultra Ultracel-10K-0.5 mL (manufactured by Merck KGaA), and the mixture was centrifuged at 12,900 rpm (150 × 100 g) and 4°C for 10 minutes to perform ultrafiltration. The flow-through solution was collected, and the amounts of n-butyric acid and lactic acid were measured by a pH buffering-electric conductivity detection method (Shimadzu high-performance liquid chromatograph, organic acid analysis system, applied data collection) using a Nexera organic acid analysis system (Shimadzu Corporation). For separating n-butyric acid and lactic acid, three Shim-pack SCR-102H (300 mm × 8 mm I.D. 7 µm) culumns were connected in series (temperature 50°C), and a 2.5 mmol/L p-toluenesulfonic acid aqueous solution (flow rate 0.8 mL/min) was used as an eluting solution. For detection, an eluate from the column was mixed with 2.5 mmol/L p-toluenesulfonic acid, 10 mmol/L Bis-Tris and a 0.05 mmol/L EDTA-4H buffer solution (flow rate 0.8 mL/min), and an electrical conductivity meter (polatity: +) was used.

Using, as an index, a value (proliferative activity) obtained by dividing the value of OD₆₀₀ of the culture solution subjected to stationary culture as described above using modified YCFA medium containing plain soy milk in a ratio of 5% (v/v), by the value of OD₆₀₀ of the culture solution (control) subjected to stationary culture as described above using modified YCFA medium free of plain soy milk, the effect of plain soy milk on proliferation of A. rectalis was evaluated. The effect of plain soy milk on the production of n-butyric acid and lactic acid was similarly evaluated using, as an index, a value (production amount ratio for each of n-butyric acid and lactic acid) obtained by dividing the value of the amount of each of n-butyric acid and lactic acid measured by the HPLC method, by the value for the control. The results of the above evaluation are shown in Table 2.

Table 2 Results of proliferative activity and production amount ratios for n-butyric acid and lactic acid in 5% (v/v) plain soy milk

**[Table 2]**

| Concentration of plain soy milk (% (v/v)) | Proliferative activity | Production amount ratio | |
|---|---|---|---|
| | | n-Butyric acid | Lactic acid |
| 0 (Control) | 1.00 | 1.00 | 1.00 |
| 5.0 | 3.74 | 2.94 | 1.94 |

## Claims

1. A butyric acid or lactic acid production enhancer comprising soy milk.

2. A food composition comprising the butyric acid or lactic acid production enhancer according to claim 1.

3. A method for promoting growth of butyrate-producing bacteria using soy milk.
